# EUROPEAN PATENT SPECIFICATION

(11) **EP 2 247 318 B1**
(45) Date of publication and mention of the grant of the patent: **16.12.2015**
(21) Application number: 09700370.1
(22) Date of filing: 02.01.2009
(51) Int. Cl.: A61L 9/04, A61L 9/012

(54) **TRANSPARENT ANHYDROUS GEL COMPRISING PERFUME**
TRANSPARENTES UND WASSERFREIES GEL BEINHALTEND PARFÜM
GEL ANHYDRE TRANSPARENT COMPORTANT DU PARFUM

(30) Priority: 04.01.2008 EP 08300007
(43) Date of publication of application: 10.11.2010
(73) Proprietor: V. Mane Fils, 06620 Bar sur Loup (FR)
(72) Inventor: STALET, Gilles, F-06130 Grasse (FR); MANE, Jean, F-06130 Grasse (FR)
(74) Representative: Dias, Sonia
(86) International application number: PCT/EP2009/050009
(87) International publication number: WO 2009/087118

(56) References cited:
- EP-A- 0 985 417
- US-A1- 2003 168 521
- US-A1- 2004 169 091

## Description

The present invention is related to a transparent anhydrous gel comprising volatile substance(s), in particular perfume and non volatile substance(s), in particular cross-linked silicone network, and not comprising filler or reinforcing agent. The invention is also related to a product comprising the gel included in a container, a method of production of the gel, the use of the gel for a controlled release of perfume in the atmosphere, a kit and the use of the kit to form the gel.

Various devices for perfuming the atmosphere are known. Such devices may simply fragrance the air, or may be used to mask bad odors. Many different types of air freshener devices have been proposed. Some of them are based on silicone elastomers, which are permeable to gases.

The perfuming devices need generally to allow controlled and substantially uniform release of a volatile organic substance such as perfume, over a broad range of working temperatures.

One particular device is described in European patent EP 1 060 751 to provide such needed article. This device comprise an article for controlled and substantially uniform release of a volatile active organic substance into the surrounding atmosphere, said article including a silicone elastomer matrix of desired shape in which are dispersed said volatile substance.

However, the article described in EP 1 060 751 comprises a limited quantity of perfume (perfume + solvent between 1 and 40% of the composition), which does not allow a long lasting use of the device, for example to perfume a large room, nor allow a very compact form of the device. Moreover, the article described in EP 1 060 751 cannot be formed extemporaneously. Thus, once the article is formed, the fragrance composition begins to be released in the atmosphere, and it will be complicate to conserve the article during a long time, with an acceptable quantity of perfume, unless an effective hermetic (and thus expensive) packaging for the article is created.

Furthermore, the composition of the article described in EP 1 060 751 does not allow to obtain a transparent product, but lead to an opaque article, which is less attractive for consumers, for esthetic reasons.

The patent publication US2004/0169091 discloses a perfumed, optionally light-transmissive gel.

There is thus a need for a device for perfuming the atmosphere, allowing uniform release of perfume, which have a long-lasting perfuming use and/or can be in a compact form with an acceptable perfuming capacity, and transparent.

The invention has for purpose to satisfy this need.

An object of the present invention is therefore a transparent anhydrous gel comprising volatile substance(s) and non volatile substance(s) characterized in that said non volatile substance(s) comprise 3.8% to 100% (by weight of non volatile substance(s)) of a cross-linked silicone network and 0 to 96.2% (by weight of non volatile substance(s)) of a non volatile solvent and said volatile substance(s) comprise 20.6 to 100% (by weight of volatile substance(s)) of perfume and 0 to 80% (by weight of volatile substance(s)) of volatile solvent, and the ratio of volatile substance / crosslinked silicone network is between 1 to 32, and said transparent anhydrous gel not comprising filler or reinforcing agent.

The term "transparent" is intended to mean a gel with a turbidity of the gel of less than 40 NTU (Nephelometric turbidity units) at 25°C, measured using a turbidimeter. "Transparent" according to the present invention does not mean "without color". Thus a transparent gel may be a transparent and coloured gel.

In a preferred embodiment of the invention, the turbidity of the gel is less than 20 NTU, more preferably less than 12 NTU, most preferably less than 8 NTU.

NTU (Nephelometric turbidity units) is the unit used to describe turbidity. The greater the scattering, the higher the turbidity. Therefore, low NTU values indicate high clarity, while high NTU values indicate low clarity.

The method of measure of a turbidimeter is based upon a comparison of the intensity of light scattered by the sample under defined conditions with the intensity of light scattered by a standard reference suspension, which is normally Turbidity-free water (distilled water or pass distilled water through a 0,45µ pore size membrane filter).

The turbidimeter shall consist of a nephelometer with light source for illuminating the sample and one or more photo-electric detectors with a readout device to indicate the intensity of light scattered at right angles to the path of the incident light. The turbidimeter should be so designed that little stray light reaches the detector in the absence of turbidity and should be free from significant drift after a short warm-up period.

The sensitivity of the instrument should preferably permit detection of a turbidity difference of 0.02 unit or less in waters having turbidities less than 1 unit. The instrument should measure from 0 to 40 units turbidity. Several ranges will be necessary to obtain both adequate coverage and sufficient sensitivity for low turbidities.

The sample tubes to be used with the available instrument must be of clear, colorless glass. They should be kept scrupulously clean, both inside and out, and discarded when they become scratched or etched. They must not be handled at all where the light strikes them, but should be provided with sufficient extra length, or with a protective case, so that they may be handled.

Differences in physical design of turbidimeters will cause differences in measured values for turbidity even though the same suspension issued for calibration. To minimize such differences, the following design criteria should be observed:
- Light source: Tungsten lamp operated at a color temperature between 2200-3000 °K.
- Distance traversed by incident light and scattered light within the sample tube: Total not to exceed 10 cm.
- Detector: Centered at 90° to the incident light path and not to exceed ± 30°C from 90°C. The Detector, and filter system if used, shall have a spectral peak response between 400 and 600 nm.

The Turbidimeter, Model 2100 and 2100 A, from the company Hach, is in wide use and has been found to be reliable; however, other instruments meeting the above design criteria are acceptable, for example the Turbi-direct® commercialized by the company Aqua lytic.

The term "anhydrous" is intended to mean "without water"; it describes a gel in which no water is present in the form of a hydrate or water of crystallization.

The term "gel" is intended to mean an elastic homogenous composition with a relative gel strength less than 20 g/mm, preferably less than 10 g/mm. The relative gel strength is measured at 25°C using a texturometer.

For example the Texturometer TA-XT from the company RHEO, can be used in the mode "measure force in compression".

An example of method of measure of relative gel strength is described below:
The gel strength is measured in a sample of 20g in a glass container in which a pressure sensor with a defined speed drops to a shallow depth in the gel.

After a trigger force of 5g is attained, the probe proceeds to penetrate into the gel to a depth of 5 mm. At this depth the maximum force reading is obtained.

The slope of the push of the freeze on the probe is measured to obtain a value gram / second or gram / mm.

Calculate the mean slope of the ascending curve by dividing the maximum force by the maximum distance give the relative relative gel strength.

It has to be noted that if the gel is too weak, it can be necessary to decrease the trigger force to 1g.

The term "volatile substance(s)" is intended to mean substance(s) which evaporate at a temperature over 25°C.

According to the present invention, volatile substance(s) comprise at least perfume. It can also comprise, optionally, volatile solvent, and other volatile substance(s) such as insecticide or an insect repellant.

The perfume can be chosen from a large number of odoriferous compounds. The perfume according to the invention can be one or a combination of several odoriferous compounds. Nonlimiting examples of these are the substances belonging to the following families:
∘ aromatic, terpene and/or sesquiterpene hydrocarbons, in particular essential oils containing these molecules and more particularly essential oils of citrus fruit (lemon, orange, grapefruit, bergamot), nutmeg, etc.,
∘ aromatic alcohols and more particularly benzyl alcohol, phenylethyl alcohol and phenylpropyl alcohol,
∘ cyclic or acyclic, saturated or unsaturated, primary, secondary or tertiary nonaromatic alcohols and more particularly linalool, citronellol, geraniol, nerol, dihydromyrcenol, terpineol and fatty alicyclic alcohols whose chain contains from 4 to 10 carbon atoms,
∘ aldehydes and more particularly saturated and unsaturated alicyclic fatty aldehydes whose carbon chain contains from 4 to 12 carbon atoms, aromatic aldehydes such as cinnamaldehyde, alpha-amylcinnamaldehyde and alpha-hexylcinnamaldehyde, lilial and phenolic aromatic aldehydes such as vanillin and ethylvanillin,
∘ phenols and more particularly aromatic phenols such as eugenol and isoeugenol, as well as methylethers thereof,
∘ carboxylic acid esters, in particular the acetic esters of benzyl alcohol, geraniol, citronellol, nerol, terpineol, borneol or linalool,
∘ aromatic acid esters such as benzoates and salicylates, as well as cinnamates esterified with alcohols of the aliphatic series containing a chain of 1 to 6 carbon atoms,
∘ aromatic phenol acids mainly in their lactone/aromatic form, such as coumarin and dihydrocoumarin,
∘ carboxylic alcohol acids in their lactone form, and more particularly the gamma octa-, gamma undeca- and gamma dodeca-lactones and the delta deca-, delta undeca- and delta dodeca-lactones in their saturated or unsaturated form,
∘ macrocyclic compounds in which the carbon chain contains from 12 to 16 carbon atoms,
∘ aromatic and/or nonaromatic ethers and acetals in their acyclic or cyclic form, and more particularly aldehyde acetals containing a carbon chain of 4 to 10 carbon atoms, as well as substituted furan and substituted or unsubstituted pyran cyclic ethers,
∘ heterocyclic compounds containing a nitrogen atom, and more particularly indole derivatives, as well as heterocyclic compounds containing 2 nitrogen atoms, and more particularly those of the pyrazine series,
∘ ketones, in particular aromatic ketones such as 4-(p-hydroxyphenyl)-2-butanone and cyclic or acyclic, saturated or unsaturated nonaromatic ketones and more particularly those of the pyrazine series,
∘ aromatic or nonaromatic sulfides, disulfides and mercaptans.

The perfume can consist of a single odoriferous compound or a mixture of such compounds.

Hesperid, floral and fruity perfume has been found to be particularly suitable.

In a preferred embodiment of the invention said perfume is at least one component selected from the group comprising aromatic, terpenic and/or sesquiterpenic hydrocarbons; aromatic alcohols; primary, secondary or tertiary, saturated or unsaturated, cyclic or acyclic nonaromatic alcohols; aldehydes; phenols; carboxylic acids; aromatic and/or nonaromatic ethers and acetals in their acyclic or cyclic form; heterocycles containing a nitrogen atom; ketones; aromatic or nonaromatic sulfides, disulfides and mercaptans, essential oils, and combinations thereof.

The volatile solvent can be chosen from a large number of solvent and can be one or a combination of several solvent. Nonlimiting examples of these are the substances belonging to the following families: Isoparaffinic solvents (Flash Point from 40 to 100°C), low molecular weight alcanes, apolar solvents.

In a preferred embodiment of the invention the volatile solvent is one or several solvents selected from the group comprising isoparaffine C7 to C12, volatile silicone Hexamethyl disiloxane, Octamethyl trisiloxane, Décamethyl tetrasiloxane, Octamethyl cyclotetrasiloxane, Decamethyl cyclopentasiloxane, Dodecamethyl cyclohexasiloxane.

More preferably, the volatile solvent is isoparaffine C10-12.

Commercial examples of volatile isoparaffins are ISOPAR C® to ISOPAR P® from EXXON CHEMICAL company, with Flash Point from 40 to 100°C.

The term "non volatile substance(s)" is intended to mean substance(s) which not evaporate at a temperature over 25°C. The non-volatile solvent can be chosen from a large number of solvent and can be one or a combination of several solvent.

In a preferred embodiment of the invention non-volatile solvent is at least one solvent selected from the group comprising:
∘ mineral oil, preferably vaseline;
∘ fatty acid ester selected in the group comprising isobutyl oleate, isopropyl oleate, butyl myristate, diisopropyl adipate (DIPA), isopropyl myristate (IPM), preferably DIPA or IPM;
∘ isoparaffines C13 to C40
∘ liquid paraffines
∘ petrolatum
and combinations thereof.

Commercial examples of mineral oil are Ecolane®60 or Ecolane®130.

Commercial examples of isoparaffins are ISOPAR V® from EXXON CHEMICAL company.

The term "cross-linked silicone network" is intended to mean a silicone resin which is formed by branched, cage-like oligosiloxanes with the general formula of RnSiXmOy, where R is a non reactive substituent, usually Me or Ph, and X is a functional group H, OH, Cl or OR. These groups are further condensed in many applications, to give highly crosslinked, insoluble polysiloxane networks.

When R is methyl, the four possible functional siloxane monomeric units are Me₃SiO, Me₂SiO₂, MeSiO₃ and SiO₄.

The cross-linked silicone network according to the present invention is obtained by reaction of a cross-linkable silicone composition, with a cross-linking agent, preferably in presence of a catalyst.

The cross-linkable silicone composition can be chosen from the various types known in the art. Cross-linkable silicone composition which are preferred are those obtained from Cross-linkable silicone compositions which cure at room temperature (known as "RTV" compositions), for example due to the effect of ambient moisture.

The term "room temperature" is intended to mean a temperature comprised between 20 and 30°C, preferably 25°C.

These RTV cross-linkable silicone are well known in the art and are commercially available from many suppliers. They are typically packaged in two parts, namely a cross-linkable silicone base and a crosslinking agent, which are mixed together at the time of use, for example in a mold. Cross-linkable silicone compositions which cure due to the effect of heating or radiation are also suitable, as are curable compositions in two or even three reactive parts.

Indeed, cross-linkable silicone compositions of the following types are suitable for the preparation of the gel of the present invention: cross-linkable silicone compositions of hydrosilylation-reaction type, cross-linkable compositions of condensation-reaction type, compositions whose cross-linking is promoted by means of organic peroxide, and compositions cross-linkable under the effect of ultraviolet radiation.

Cross-linkable silicone compositions of a hydrosilylation reaction type are most suitable.

For example, a cross-linkable silicone composition of hydrosilylation-reaction type may consist at least of the following components: an organopolysiloxane with at least two alkenyl groups in one molecule, an organopolysiloxane with at least two silicone-bonded hydrogen atoms in one molecule, and a platinum-type catalyst.

A condensation-reaction type cross-linkable silicone composition may consist, e. g., at least of the following components: an organopolysiloxane having in one molecule hydroxyl groups bonded to silicon atoms or at least two hydrolysable groups such as an aminoxy groups, acetoxy groups, oxime groups, alkoxy groups, or hydroxyl groups bonded to silicon atoms; a silane-type cross-linking agent having in one molecule at least three hydrolysable groups, such as aminoxy groups, acetoxy groups, oxime groups, or alkoxy groups bonded to silicon atoms; and a condensation-reaction catalyst such as an organotin compound, an organotitanium compound, or the like.

Most preferable is a de-alcoholization condensation-reaction type cross-linkable silicone composition consisting at least of an organopolysiloxane having in one molecule at least two silicon-bonded hydroxyl groups or alkoxy groups, a silane-type cross-linking agent having in one molecule at least three silicon-bonded alkoxy groups, and a condensation-reaction catalyst such as an organotin compound, an organotitanium compound, or the like.

The aforementioned compounds can be additionally combined with the following components: 3-glycidoxypropyltrimethoxysilane, 3-glycidoxypropylmethyldimethoxysilane, 2- (3, 4- epoxycyclohexyl) ethyl trimethoxysilane, 3-methacryloxypropyltrimethoxysilane, 3-methacryloxypropyltriethoxysilane, N- (2-aminoethyl)-3-aminopropyltrimethoxysilane, or a similar organic functional alkoxysilane.

Cross-linkable silicone compositions of a hydrosilylation reaction type are preferred according to the present invention. In particular, cross-linkable silicones according to the present invention are functionalized silicone polymer.

In a preferred embodiment of the invention, the function of said functionalized silicone polymers is selected from the group comprising vinyl group, allyl group, acryl group, hydroxyl group, and Si-H group.

In a preferred embodiment of the invention, said functionalized silicone polymers is selected from the group comprising hydroxyl-end blocked polydimethylsiloxane, preferably with a viscosity comprised between 40 and 3 000 000 mm²/s.

In a preferred embodiment of the invention cross-linking agent is tetra-alkoxysilane or silane.

In a preferred embodiment of the invention catalyst is titanium platinum or tin, preferably tin.

In a preferred embodiment of the invention the ratio (in weight) of volatile substance / crosslinked silicone network is between 7 to 22.

The terms "filler" or "reinforcing agent" is intended to mean a substance that increases the toughness, abrasion resistance, tear resistance and strength of the gel when added to it in substantial quantities. Finely divided powders such as carbon black, zinc oxide, calcium carbonate and silica greatly enhance these properties in silicon rubber and other elastomers, and permit to obtain in particular one piece- gel like composition, which is the contrary of the aim of the present invention.

In a preferred embodiment of the invention, non volatile substance(s) comprise also 0 to 2% (by weight of non volatile substance(s)) of at least a compound which is non-volatile, soluble in volatile substance(s) and not soluble in the functionalized silicone polymers used for the preparation of the gel and which will be cross-linked by a gelling agent to lead to said cross-linked silicone polymers, and combinations thereof.

More preferably, said compound is selected from the group comprising dipropylene glycol, propylene glycol, benzyle benzoate, diethyl phtalate, triethyle citrate, preferably dipropylene glycol or triethyl citrate.

The aim of said compound is to turn the gel to opaque when all the volatile substance(s) of the gel are evaporated. It is the signal for the consumer that the gel is now not effective to perfume the atmosphere, and need to be changed.

In a preferred embodiment of the invention, non volatile substance(s) comprise also 0 to 1% (by weight of non volatile substance(s)) of a colouring agent.

In a preferred embodiment of the invention, the transparent anhydrous gel comprises:

| | |
|---|---|
| perfume | 20% to 96.975%, preferably 20 to 59 %, more preferably 29 to 59 %, even more preferably 39 to 59%. |
| cross-linked silicone network | 3.025% to 18.5%, preferably 8 to 13 % |
| volatile solvent | 0 to 76.975% preferably 29 to 68 % |
| non Volatile solvent | 0 to 76.975% |

According to the present invention, a preferred example of transparent anhydrous gel consists of:
functionalized silicone polymer (in particular Hydroxy-endblocked polydimethylsiloxane)= 8,28%
volatile solvent (in particular Isoparaffin ISOPAR G) = 50.84%
perfume = 39.41%
gelling agent (tetraethoxysilane = 73%; Dibutyltin Dilaurate = 27%)= 1.48%
colouring agent (in particular Liposoluble Black Dye) = 0.001%

Weight and mix in a large blend tank until homogeneous, then pack in individual containers before gelification occurs. The parameters of this gel are indicated below:
Transparency = 6.90 NTU
Resistance to compression = 1.3 g/mm

A second object of the present invention is a product comprising a transparent anhydrous gel according to the invention, included in a container.

The term "included in a container" is intended to mean that the gel is not a one piece gel-like composition, which can be considered as a solid, but a gel with strength less than 20 g/mm, preferably less than 10 g/mm, that need to be in a packaging to be stored or used.

The container can be for example in plastic or glass, in different shape or size, with a device for opening, in order to expose the surface of the gel with the atmosphere to perfume.

A third object of the present invention is a method of production of a transparent anhydrous gel according to the invention, characterized in that it comprises the following steps:
(a) mixing volatile substance(s) and non volatile substance(s), said non volatile substance(s) comprise 3.77 % to 100% (by weight of non volatile substance(s)) of a functionalized silicone polymers and 0 to 96.23% (by weight of non volatile substance(s)) of a non volatile solvent and said volatile substance(s) comprise 20.6 to 100% (by weight of volatile substance(s)) of perfume and 0 to 80% (by weight of volatile substance(s)) of volatile solvent, and the ratio of volatile substance / functionalized silicone polymers is between 1 to 32;
(b) adding a gelling agent to the mix obtained after step (a), said gelling agent being 0,025 to 3,5 % by weight of the mix obtained after step (a);
no filler or reinforcing agent being added in step (a) or(b).

In a preferred embodiment of the invention, the function of said functionalized silicone polymers is selected from the group comprising vinyl group, allyl group, acryl group, hydroxyl group, and Si-H group.

In a preferred embodiment of the invention, said functionalized silicone polymers is selected from the group comprising hydroxyl-end blocked polydimethylsiloxane, preferably with a viscosity comprised between 40 and 3 000 000 mm²/s.

In a preferred embodiment of the invention, steps of the method, in particular step (b), are realized at room temperature.

The term "room temperature" is intended to mean a temperature comprised between 20 and 30°C, preferably 25°C.

In a preferred embodiment of the invention, 0,025 to 3,5 % by weight of the mix obtained after step (a) of the gelling agent consists of:
∘ 0,2 to 2,5 % by weight of the mix obtained after step (a) of cross-linking agent, preferably tetra-alkoxysilane or silane;
∘ 0,05 to 1% by weight of the mix obtained after step (a) of catalyst, preferably titanium platinum or tin, more preferably tin.

A fourth object of the present invention is the use of a transparent anhydrous gel according to the present invention, or a product according to the present invention, for a controlled release of perfume into the surrounding atmosphere by contact of a surface of the gel with the atmosphere.

One advantage of the gel of the invention is its capacity of containing important quantities of perfume with respect to its overall composition. It is therefore particularly suitable for use in air fresheners, such as room air fresheners and car air fresheners (also called carfresheners). When used as car air freshener other advantages of the gel of the invention are exploited. In fact, in contrast to many state of the art gel based air fresheners, the gel of the invention is temperature resistant, i.e. it is neither altered at low temperatures, especially temperatures below 0°C, since it does not contain any water nor does it melt away at high temperatures, e.g. in the sun. Moreover, the use of a non volatile solvent renders the gel non-flammable, which presents a major safety argument.

Among others, due to its capacity of containing important quantities of perfume with respect to its overall composition, the gel of the invention may also advantageously be integrated into the packaging of another product in order to provide for example the perfume of the product contained in the packaging, thus avoiding that the consumer opens the same before purchasing.

Another advantage of the gel according to the invention is that it is anhydrous and inert with respect to water. It does therefore not dissolve or disintegrate when it comes into contact wit a humid atmosphere or water. Particular interesting applications of the transparent anhydrous gel according to the invention or the product according to the invention for a controlled release of perfume into the surrounding atmosphere by contact of a surface of the gel with the atmosphere are thus applications in which the surrounding atmosphere is a humid atmosphere. Such applications include but are not limited to air fresheners for damp rooms, such as bath rooms, shower cubicles, and restrooms. The transparent anhydrous gel according to the invention may also be used as perfuming element in solid, liquid or gel deodorants for water-closets.

Due to its inertness with respect to water, the transparent anhydrous gel according to the invention may also be used for the manufacture of perfuming articles for use in household cleaning appliances, such as dish washers, washing machines, and dryers.

In another application, the transparent anhydrous gel according to the invention is used as perfumed coating on a substrate. Suitable substrates include porous substrates, such as ceramics, tiles, and cellulose, and non porous substrates such as glass and plastics.

The transparent anhydrous gel according to the invention is also useful as impregnation system for tissues and fibers. In this case, the liquid formulation containing all the ingredients of the gel of the invention is preferably applied before gelling to the tissue or fiber(s) by spraying or immersion.

In another application, the transparent anhydrous gel according to the invention is used as perfuming system for powders. In this case, the liquid formulation containing all the ingredients of the gel of the invention is preferably applied before gelling to the powder by spaying.

A fifth object of the present invention is a kit to obtain a transparent anhydrous gel according to the present invention comprising:
(i) volatile substance(s) and non volatile substance(s), said non volatile substance(s) comprise 3.77 % to 100% (by weight of non volatile substance(s)) of a functionalized silicone polymers and 0 to 96.23% (by weight of non volatile substance(s)) of a non volatile solvent and said volatile substance(s) comprise 20.6 to 100% (by weight of volatile substance(s)) of perfume and 0 to 80% (by weight of volatile substance(s)) of volatile solvent, and the ratio of volatile substance / functionalized silicone polymers is between 1 to 32;
(ii) gelling agent.
where (ii) is 0,025 to 3,5 % by weight of (i), (ii) is provided separately from the mix (i), and the kit does not comprise filler or reinforcing agent.

In a preferred embodiment of the invention the kit according to the present invention, allow to obtain a transparent anhydrous gel according to the present invention by mixture of (i) and (ii).

### EXAMPLES

The following examples describe preferred embodiments of the transparent anhydrous gel according to this invention, without however limiting the scope.

### Example 1: COMPOSITION OF THE GELLING AGENT

TETRAETHOXYSILANE = 73%
Dibutyltin Dilaurate = 27%

### Example 2: SOLVENT-LESS FRAGRANCED GEL

### PHASE A

1- Hydroxy-endblocked polydimethylsiloxane (DOW CORNING(R) 3-0084 14,000 CS POLYMER) = 10g (9.910%)
2- PERFUME = 90g (89.188%)
3- Liposoluble blue dye = 0.01g (0.001%)
Mix until polymer is fully dispersed in fragrance
4- Add Example 1 GELLING AGENT = 0.9g (0.891%)

Add to Phase A, mix until homogeneous and pour into a container of glass or PET, close and let gel horizontally for 4 hours.
Transparency value is 6.54 NTU
Resistance to Compression = 0.08 g/mm

**Example 3:** TYPICAL FORMULATION

Weight and mix in a large blend tank until homogeneous, then pack in individual containers before gelification occurs.
1- Hydroxy-endblocked polydimethylsiloxane (DOW CORNING(R) 3-0084 14,000 CS POLYMER) = 8,28%
2- ISOPAR G (Exxon) = 50.84%
3- PERFUME = 39.41%
4- GELLING AGENT = 1.48%
5- Liposoluble Black Dye = 0.001%

Transparency = 6.90 NTU
Resistance to compression = 1.3 g/mm

### Example 4: FORMULATION WITH NON-VOLATILE SOLVENT

Weight and mix in a large blend tank until homogeneous, then pack in individual containers before gelification occurs.
1- Hydroxy-endblocked polydimethylsiloxane (DOW CORNING(R) 3-0084 14,000 CS POLYMER) = 9,8%
2- ECOLANE 130 (Total) = 39.22%
3- PERFUME = 49.02%
4- GELLING AGENT = 1.96%

### Example 5: FRAGRANCE DIFFUSION

### Formulations with volatile solvents

| Example Nb | RUN# | Isoparafin Flash Point (°C, closed cup) | Perfume | Solvent | Polymer | Gelling agent |
|---|---|---|---|---|---|---|
| 5 | 1 | 40 | 59% | 29% | 11% | 1,48% |
| 6 | 4 | 40 | 20% | 68% | 11% | 1,48% |
| 7 | 5 | 60 | 39% | 53% | 6% | 2,46% |
| 8 | 7 | 100 | 59% | 29% | 11% | 1,48% |
| 9 | 8 | 100 | 20% | 68% | 11% | 1,97% |

### Fragrance Delivery Results

| Example Nb | RUN# | Turbidity (NTU) | Relative Gel Strength (g/mm) | Weight loss 10 days (%) | Weight loss 30 days (%) | Weight loss 45 days (%) |
|---|---|---|---|---|---|---|
| 5 | 1 | 11.8 | 1.6 | 29,7 | 59,1 | 71.4 |
| 6 | 4 | 2.4 | 2.8 | 43,6 | 88,6 | 95.5 |
| 7 | 5 | 6.2 | 0.55 | 22,2 | 52,6 | 65.5 |
| 8 | 7 | 9.3 | 1.7 | 19,2 | 37,8 | 44.1 |
| 9 | 8 | 1.6 | 2.8 | 9,7 | 17,9 | 78.6 |

### Other formulations with non volatiles solvents (release profiles):

| Example Nb | Perfumes label | Perfume % | Ecolane 130 | Polymer | Gelling agent |
|---|---|---|---|---|---|
| 10 | PEACH BLOSSOM | 49% | 39% | 9.8% | 2.2% |
| 11 | GOLDEN GARDEN | 49% | 39% | 9.8% | 2.2% |
| 12 | ANTI TOBBACCO | 49% | 39% | 9.8% | 2.2% |
| 13 | SEA MINT | 49% | 39% | 9.8% | 2.2% |
| 14 | GREEN TEA | 49% | 39% | 9.8% | 2.2% |

### Fragrance Delivery Results

| Example Nb | Weight loss 10 days (%) | Weight loss 30 days (%) | Weight loss 45 days (%) |
|---|---|---|---|
| 10 | 17% | 29% | 47% |
| 11 | 25% | 42% | 51% |
| 12 | 30% | 49% | 56% |
| 13 | 25% | 43% | 50% |
| 14 | 13% | 25% | 47% |

### Example 6: FORMULATION WITH 1% OF NON-VOLATILE, SOLUBLE IN VOLATILE SUBSTANCE(S) AND NOT SOLUBLE IN THE FUNCTIONALIZED SILICONE POLYMERS COMPOUND

The composition comprises 1% of a non-volatile, soluble in volatile substance(s) and not soluble in the functionalized silicone polymers compounds chosen between dipropylene glycol, triethyl citrate, diethyl phtalate, benzyl benzoate, in an amount of 1% of the total weight of the composition.

These compounds are named "end of life" agent because the aim of said compounds is to turn the gel to opaque when all the volatile substance(s) of the gel are evaporated. It is the signal for the consumer that the gel is now not effective to perfume the atmosphere, and need to be changed (product is in the end of life).

The ingredients of the composition are weighted and mixed in a large blend tank until homogeneous, then pack in individual containers before gelification occurs.

Composition of the transparent anhydrous gel:
1- Hydroxy-endblocked polydimethylsiloxane (DOW CORNING(R) 3-0084 14,000 CS POLYMER) = 8,05%
2- ISOPAR G (Exxon) = 49.45%
3- PERFUME = 40.00%
4- END OF LIFE AGENT = 1,00%
5- GELLING AGENT = 1.50%

After a 5 week diffusion time, the gel turns opaque thanks to the end of life agent and because all the volatile substance(s) of the gel are evaporated. The gel turbidity is estimated by comparison to a set of standards:
dipropylene glycol > 200 NTU
triethyl citrate > 200 NTU
diethyl phtalate > 400 NTU
benzyl benzoate > 800 (less than 1000) NTU

### Example 7: FORMULATION WITH 2% OF NON-VOLATILE, SOLUBLE IN VOLATILE SUBSTANCE(S) AND NOT SOLUBLE IN THE FUNCTIONALIZED SILICONE POLYMERS COMPOUND

The composition comprises 2% of a non-volatile, soluble in volatile substance(s) and not soluble in the functionalized silicone polymers compounds chosen between dipropylene glycol, triethyl citrate, diethyl phtalate, benzyl benzoate, in an amount of 1% of the total weight of the composition.

The ingredients of the composition are weighted and mixed in a large blend tank until homogeneous, then pack in individual containers before gelification occurs.

Composition of the transparent anhydrous gel:
1- Hydroxy-endblocked polydimethylsiloxane (DOW CORNING(R) 3-0084 14,000 CS POLYMER) = 7.91 %
2- ISOPAR G (Exxon) = 45.59%
3- PERFUME = 40.00%
4- END OF LIFE AGENT = 2,00%
5- GELLING AGENT = 1.50%

After a 6 week diffusion time, the gel turns opaque thanks to the end of life agent and because all the volatile substance(s) of the gel are evaporated. The gel turbidity is estimated by comparison to a set of standards:
dipropylene glycol > 200 NTU
triethyl citrate > 200 NTU
diethyl phtalate > 400 NTU
benzyl benzoate > 800 (less than 1000) NTU

### Example 8: PERFUME EXAMPLES

Compositions of transparent anhydrous gel of example 1 to 7 are tested with success with various perfumes, as citrus perfume and clean linen perfume.

The composition of citrus perfume and clean linen perfume are given below:

| **CITRUS PERFUME** | |
|---|---|
| LIMONENE | 62.6% |
| BENZYL ACETATE | 5.0% |
| DIHYDROMYRCENOL | 5.0% |
| 3,7-DIMETHYL-2,6-OCTADIENAL (CIS & TRANS ISOMERS) | 5.0% |
| BERGAMYL ACETATE | 4.0% |
| LINALYL ACETATE | 4.0% |
| TETRAHYDROLINALOL | 4.0% |
| GERANYL ACETATE | 2.0% |
| HEXYL ACETATE | 2.0% |
| CITRONELLYL ACETATE | 1.0% |
| LEMON ESSENTIAL OIL | 1.0% |
| 3-METHYL-5-PHENYLPENT-2-ENENITRILE | 1.0% |
| ISOAMYL ACETATE | 0.5% |
| PROPENYL HEXANOATE | 0.5% |
| ETHYL HEXANOATE | 0.5% |
| 2,4,6-METHYL-4-PHENYL-1,3-DIOXANE | 0.5% |
| CITRONELLYL FORMIATE | 0.5% |
| ETHYL BUTYRATE | 0.2% |
| GERANIUM ESSENTIAL OIL | 0.2% |
| TRANS 2 DODECENAL | 0.1% |
| L-CARVONE | 0.1% |
| CIS-3-METHYL-2-(2-PENTENYL)-2-CYCLOPENTEN-1-ONE | 0.1% |
| 3-HYDROXY-2-ETHYL-4-PYRONE | 0.1% |
| PARAMENTHENETHIOL | 0.1% |

| **CLEAN LINEN PERFUME** | |
|---|---|
| LINALYL ACETATE | 22,60% |
| BENZYL ACETATE | 7,92% |
| DIHYDROMYRCENOL | 14,14% |
| ISOBORNYL ACETATE | 18,10% |
| CITRATHAL | 6,79% |
| ISOBORNEOL | 4,53% |
| MINT ESSENTIAL OIL | 3,39% |
| ETHYLE ACETATE | 2,83% |
| HEXYL ACETATE | 4,53% |
| STYRALLYL ACETATE | 2,83% |
| EUCALYPTOL | 1,70% |
| 2-METHYL UNDECANAL | 1,13% |
| 3 -(O- AND P-ETHYLPHENYL)-2,2-DIMETHYLPROPIONALDEHYDE | 1,13% |
| ISOMENTHONE | 1,13% |
| MENTHONE | 1,13% |
| 5-METHYL-3-HEPTANONE | 0,91% |
| ISOAMYL ACETATE | 0,68% |
| ALPHA IONONE | 0,68% |
| DODECANAL | 0,57% |
| 1-(5,5-DIMETHYL-1-CYCLOHEXEN-1-YL)-4-PENTEN-1-ONE | 0,57% |
| PATCHOULY ESSENTIAL OIL | 0,57% |
| 1-METHYL-3-(4-METHYL-3-PENTYL)-3-CYCLOHEXENE-1-CARBOXALDEHYDE | 0,57% |
| CIS 3 HEXENYL ACETATE | 0,23% |
| ACETYL METHYL ANTHRANILATE | 0,23% |
| LAVANDIN ESSENTIAL OIL | 0,11% |
| SPEARMINT ESSENTIAL OIL | 0,11% |
| 2-HEPTANONE | 0,23% |
| 2-OCTANONE | 0,23% |
| P-METHYL ISOPROPYL BENZENE | 0,11% |
| ALLYL HEPTOATE | 0,11% |
| PARAMENTHENETHIOL | 0,06% |
| GALBANUM ESSENTIAL OIL | 0,05% |
| ISOBUTYL QUINOLEIN | 0,06% |
| OXANE | 0,02% |
| TRANS 2 DODECENAL | 0,02% |

## Claims

1. Transparent anhydrous gel with a relative gel strength less than 20 g/mm, comprising volatile substance(s) and non volatile substance(s), wherein said non volatile substance(s) comprise 3.8% to 100% (by weight of non volatile substance(s)) of a cross-linked silicone network and 0 to 96.2% (by weight of non volatile substance(s)) of a non volatile solvent and said volatile substance(s) comprise 20.6 to 100% (by weight of volatile substance(s)) of perfume and 0 to 80% (by weight of volatile substance(s)) of volatile solvent, and the ratio of volatile substance / crosslinked silicone network is between 1 to 32, and said transparent anhydrous gel not comprising filler or reinforcing agent.

2. Transparent anhydrous gel according to claim 1, **characterized in that** the volatile solvent is one or several solvents selected from the group comprising isoparaffine C7 to C12, volatile silicone Hexamethyl disiloxane, Octamethyl trisiloxane, Décamethyl tetrasiloxane, Octamethyl cyclotetrasiloxane, Decamethyl cyclopentasiloxane, Dodecamethyl cyclohexasiloxane.

3. Transparent anhydrous gel according to claim 2, **characterized in that** the volatile solvent is isoparaffine C10-12.

4. Transparent anhydrous gel according to any of claims 1 to 3, **characterized in that** the non-volatile solvent is at least one solvent selected from the group comprising:
∘ mineral oil, preferably vaseline;
∘ fatty acid ester selected in the group comprising isobutyl oleate, isopropyl oleate, butyl myristate, diisopropyl adipate (DIPA), isopropyl myristate (IPM), preferably DIPA or IPM;
∘ isoparaffines C13 to C40;
∘ liquid paraffines;
∘ petrolatum;
and combinations thereof.

5. Transparent anhydrous gel according to any of claims 1 to 4, **characterized in that** non volatile substance(s) comprise 0 to 2% (by weight of non volatile substance(s)) of at least a compound which is non-volatile, soluble in volatile substance(s) and not soluble in the functionalized silicone polymers used for the preparation of the gel and which will be cross-linked by a gelling agent to lead to said cross-linked silicone polymers, and combinations thereof.

6. Transparent anhydrous gel according to claim 5, **characterized in that** said compound is selected from the group comprising dipropylene glycol, propylene glycol, benzyle benzoate, diethyl phtalate, triethyle citrate, preferably dipropylene glycol or triethyl citrate.

7. Transparent anhydrous gel according to any of claims 1 to 6, **characterized in that** the turbidity of the gel is less than 20 NTU, preferably less than 12 NTU, most preferably less than 8 NTU.

8. Transparent anhydrous gel according to any of claims 1 to 7, **characterized in that** the relative gel strength of the gel is less than 10 g/mm.

9. Product comprising a transparent anhydrous gel according to any of claims 1 to 8, included in a container.

10. Method of production of a transparent anhydrous gel with a relative gel strength less than 20 g/mm, according to any of claims 1 to 8, comprising the following steps:
(a) mixing volatile substance(s) and non volatile substance(s), said non volatile substance(s) comprise 3.77 % to 100% (by weight of non volatile substance(s)) of a functionalized silicone polymers and 0 to 96.23% (by weight of non volatile substance(s)) of a non volatile solvent and said volatile substance(s) comprise 20.6 to 100% (by weight of volatile substance(s)) of perfume and 0 to 80% (by weight of volatile substance(s)) of volatile solvent, and the ratio of volatile substance / functionalized silicone polymers is between 1 to 32;
(b) adding a gelling agent to the mix obtained after step (a), said gelling agent being 0,025 to 3,5 % by weight of the mix obtained after step (a);
no filler or reinforcing agent being added in step (a) or(b).

11. Method according to claim 10, **characterized in that** the function of said functionalized silicone polymers is selected from the group comprising vinyl group, allyl group, acryl group, hydroxyl group, and Si-H group.

12. Method according to claim 10 or 11, **characterized in that** said functionalized silicone polymers is selected from the group comprising hydroxyl-end blocked polydimethylsiloxane, preferably with a viscosity comprised between 40 and 3 000 000 mm²/s.

13. Method according to any of claims 10 to 12, **characterized in that** steps, in particular step (b), are realized at room temperature.

14. Method according to any of claims 11 to 13, **characterized in that** 0,025 to 3,5 % by weight of the mix obtained after step (a) of the gelling agent consists of:
∘ 0,2 to 2,5 % by weight of the mix obtained after step (a) of cross-linking agent, preferably tetra-alkoxysilane or silane;
∘ 0,05 to 1% by weight of the mix obtained after step (a) of catalyst, preferably titanium platinum or tin, more preferably tin.

15. Kit to obtain a transparent anhydrous gel with a relative gel strength less than 20 g/mm, according to the present invention according to any of claims 1 to 8, comprising:
(i) volatile substance(s) and non volatile substance(s), said non volatile substance(s) comprise 3.77 % to 100% (by weight of non volatile substance(s)) of a functionalized silicone polymers and 0 to 96.23% (by weight of non volatile substance(s)) of a non volatile solvent and said volatile substance(s) comprise 20.6 to 100% (by weight of volatile substance(s)) of perfume and 0 to 80% (by weight of volatile substance(s)) of volatile solvent, and the ratio of volatile substance / functionalized silicone polymers is between 1 to 32;
(ii) gelling agent.
where (ii) is 0,025 to 3,5 % by weight of (i), (ii) is provided separately from the mix (i), and the kit does not comprise filler or reinforcing agent.

## Patentansprüche

1. Transparentes wasserfreies Gel mit einer relativen Gelfestigkeit von weniger als 20 g/mm, das flüchtige Substanz(en) und nicht-flüchtige Substanz(en) umfasst, wobei die nicht-flüchtige Substanz(en) 3,8% bis 100% (nach Gewicht, bezogen auf die nicht-flüchtige(n) Substanz(en)) eines vernetzten Siliconnetzwerks und 0 bis 96,2% (nach Gewicht, bezogen auf die nicht-flüchtige(n) Substanz(en)) eines nicht flüchtigen Lösungsmittels umfasst (umfassen) und wobei die flüchtige(n) Substanz(en) 20,6 bis 100% (nach Gewicht, bezogen auf die flüchtige(n) Substanz(en)) Parfüm und 0 bis 80% (nach Gewicht, bezogen auf die flüchtige(n) Substanz(en)) flüchtiges Lösungsmittel umfasst (umfassen), und wobei das Verhältnis von flüchtiger Substanz / vernetztem Siliconnetzwerk zwischen 1 und 32 liegt und das transparente wasserfreie Gel keinen Füllstoff und kein Vernetzungsmittel umfasst.

2. Transparentes wasserfreies Gel gemäß Anspruch 1, **dadurch gekennzeichnet, dass** das flüchtige Lösungsmittel ein Lösungsmittel oder mehrere Lösungsmittel, ausgewählt aus der Gruppe, umfassend C7- bis C12-Isoparaffin, flüchtiges Siliconhexamethyldisiloxan, Octamethyltrisiloxan, Decamethyltetrasiloxan, Octarnethylcyclotetrasiloxan, Decamethylcyclopentasiloxan, Dodecamethylcyclohexasiloxan, ist.

3. Transparentes wasserfreies Gel gemäß Anspruch 2, **dadurch gekennzeichnet, dass** das flüchtige Lösungsmittel C10-12-Isoparaffin ist.

4. Transparentes wasserfreies Gel gemäß einem der Ansprüche 1 bis 3, **dadurch gekennzeichnet, dass** das nicht-flüchtige Lösungsmittel wenigstens ein Lösungsmittel ist, das aus der Gruppe, umfassend:
∘ Mineralöl, vorzugsweise Vaseline;
∘ Fettsäureester, ausgewählt in der Gruppe, umfassend Isobutyloleat, Isopropyloleat, Butylmyristat, Diisopropyladipat (DIPA), Isopropylmyristat (IPM), vorzugsweise DIPA oder IPM;
∘ C13- bis C40-Isoparaffine;
∘ flüssige Paraffine;
∘ Petrolatum;
und Kombinationen davon, ausgewählt ist.

5. Transparentes wasserfreies Gel gemäß einem der Ansprüche 1 bis 4, **dadurch gekennzeichnet, dass** die nicht-flüchtige Substanz (die nicht-flüchtigen Substanzen) 0 bis 2% (nach Gewicht, bezogen auf die nicht-flüchtige(n) Substanz(en)) wenigstens einer Verbindung, die nicht flüchtig ist, in der flüchtigen Substanz (den flüchtigen Substanzen) löslich ist und in den funktionalisierten Siliconpolymeren, die für die Herstellung des Gels verwendet werden und die durch ein Geliermittel vernetzt werden, um zu den vernetzten Siliconpolymeren zu gelangen, nicht löslich ist, und Kombinationen davon umfasst (umfassen).

6. Transparentes wasserfreies Gel gemäß Anspruch 5, **dadurch gekennzeichnet, dass** die Verbindung aus der Gruppe, umfassend Dipropylenglycol, Propylenglycol, Benzylbenzoat, Diethylphtalat, Triethylcitrat, vorzugsweise Dipropylenglycol oder Triethylcitrat, ausgewählt ist.

7. Transparentes wasserfreies Gel gemäß einem der Ansprüche 1 bis 6, **dadurch gekennzeichnet, dass** die Trübung des Gels weniger als 20 NTU, vorzugsweise weniger als 12 NTU, am bevorzugtesten weniger als 8 NTU ist.

8. Transparentes wasserfreies Gel gemäß einem der Ansprüche 1 bis 7, **dadurch gekennzeichnet, dass** die relative Gelfestigkeit des Gels weniger als 10 g/mm ist.

9. Produkt, das ein transparentes wasserfreies Gel gemäß einem der Ansprüche 1 bis 8, das in einem Behälter enthalten ist, umfasst.

10. Verfahren zur Herstellung eines transparenten wasserfreien Gels mit einer relativen Gelfestigkeit von weniger als 20 g/mm gemäß einem der Ansprüche 1 bis 8, umfassend die folgenden Schritte:
(a) Mischen einer flüchtigen Substanz (flüchtiger Substanzen) und einer nicht-flüchtigen Substanz (nicht-flüchtiger Substanzen), wobei die nicht-flüchtige Substanz (die nicht flüchtigen Substanzen) 3,77% bis 100% (nach Gewicht, bezogen auf die nicht-flüchtige(n) Substanz(en)) eines funktionalisierten Siliconpolymers und 0 bis 96,23% (nach Gewicht, bezogen auf die nicht-flüchtige(n) Substanz(en)) eines nicht-flüchtigen Lösungsmittels umfasst (umfassen) und die flüchtige Substanz (die flüchtigen Substanzen) 20,6 bis 100% (nach Gewicht, bezogen auf die flüchtige(n) Substanz(en)) Parfüm und 0 bis 80% (nach Gewicht, bezogen auf die flüchtige(n) Substanz(en)) flüchtiges Lösungsmittel umfasst (umfassen) und wobei das Verhältnis von flüchtiger Substanz / funktionalisierten Siliconpolymeren zwischen 1 und 32 liegt;
(b) Zusetzen eines Geliermittels zu der nach Schritt (a) erhaltenen Mischung, wobei das Geliermittel 0,025 bis 3,5 Gewichts-% der in Schritt (a) erhaltenen Mischung ist;
wobei kein Füllstoff und kein Verstärkungsmittel in Schritt (a) oder (b) zugesetzt werden.

11. Verfahren gemäß Anspruch 10, **dadurch gekennzeichnet, dass** die Funktion der funktionalisierten Siliconpolymere aus der Gruppe, umfassend eine Vinylgruppe, eine Allylgruppe, eine Acrylgruppe, eine Hydroxylgruppe und eine Si-H-Gruppe, ausgewählt ist.

12. Verfahren gemäß Anspruch 10 oder 11, **dadurch gekennzeichnet, dass** die funktionalisierten Siliconpolymere aus der Gruppe, umfassend am Hydroxylende blockiertes Polydimethylsiloxan, vorzugsweise mit einer Viskosität zwischen 40 und 3.000.000 mm²/s, ausgewählt werden.

13. Verfahren gemäß einem der Ansprüche 10 bis 12, **dadurch gekennzeichnet, dass** Schritte, insbesondere Schritt (b), bei Raumtemperatur durchgeführt werden.

14. Verfahren gemäß einem der Ansprüche 11 bis 13, **dadurch gekennzeichnet, dass** 0,025 bis 3,5 Gewichts-% der in Schritt (a) erhaltenen Mischung an Geliermittel aus Folgendem bestehen:
∘ 0,2 bis 2,5 Gewichts-% der in Schritt (a) erhaltenen Mischung an Vernetzungsmittel, vorzugsweise Tetraalkoxysilan oder Silan;
∘ 0,05 bis 1 Gewichts-% der in Schritt (a) erhaltenen Mischung an Katalysator, vorzugsweise Titan, Platin oder Zinn, bevorzugter Zinn.

15. Kit zur Herstellung eines transparenten wasserfreien Gels mit einer relativen Gelfestigkeit von weniger als 20 g/mm gemäß der vorliegenden Erfindung gemäß einem der Ansprüche 1 bis 8, umfassend:
(i) flüchtige Substanz(en) und nicht-flüchtige Substanz(en), wobei die nicht-flüchtige(n) Substanz(en) 3,77% bis 100% (nach Gewicht, bezogen auf die nicht-flüchtige(n) Substanz(en)) funktionalisierte Siliconpolymere und 0 bis 96,23% (nach Gewicht, bezogen auf die nicht-flüchtige(n) Substanz(en)) eines nicht-flüchtigen Lösungsmittels umfasst (umfassen) und wobei die flüchtige(n) Substanz(en) 20,6 bis 100% (nach Gewicht, bezogen auf die flüchtige(n) Substanz(en)) Parfüm und 0 bis 80% (nach Gewicht, bezogen auf die flüchtige(n) Substanz(en)) flüchtiges Lösungsmittel umfasst (umfassen) und wobei das Verhältnis von flüchtiger Substanz / funktionalisierten Siliconpolymeren zwischen 1 und 32 liegt;
(ii) Geliermittel,
wobei (ii) 0,025 bis 3,5 Gewichts-% von (i) ist, (ii) getrennt von der Mischung (i) bereitgestellt wird und der Kit keinen Füllstoff und kein Verstärkungsmittel umfasst.

## Revendications

1. Gel anhydre transparent présentant une résistance de gel relative inférieure à 20 g/mm, comprenant une ou plusieurs substances volatiles et une ou plusieurs substances non volatiles dans lequel lesdites une ou plusieurs substances non volatiles comprennent 3,8 % à 100 % (en poids des substances non volatiles) d'un réseau de silicone réticulé et 0 à 96,2 % (en poids des substances non volatiles) d'un solvant non volatil et lesdites une ou plusieurs substances volatiles comprennent 20,6 à 100 % (en poids des substances volatiles) de parfum et 0 à 80 % (en poids des substances volatiles) de solvant volatil, et le rapport de substance volatile / réseau de silicone réticulé est compris entre 1 et 32, et ledit gel anhydre transparent ne comprend pas de charge ou d'agent renforçant.

2. Gel anhydre transparent selon la revendication 1, **caractérisé en ce que** le solvant volatil est un ou plusieurs solvants choisis dans le groupe comprenant l'isoparaffine C7 à C12, l'hexaméthyle disiloxane de silicone volatile, l'octaméthyle trisiloxane, le décaméthyle tétrasiloxane, l'octaméthyle cyclotétrasiloxane, le décaméthyle cyclopentasiloxane, le dodécaméthyle cyclohexasiloxane.

3. Gel anhydre transparent selon la revendication 2, **caractérisé en ce que** le solvant volatil est l'isoparaffine C10-12.

4. Gel anhydre transparent selon l'une quelconque des revendications 1 à 3, **caractérisé en ce que** le solvant non volatil est au moins un solvant choisi dans le groupe comprenant :
- une huile minérale, de préférence la vaseline ;
- un ester d'acide gras choisi dans le groupe comprenant l'oléate d'isobutyle, l'oléate d'isopropyle, le myristate de butyle, l'adipate de diisopropyle (DIPA), le myristate d'isopropyle (IPM), de préférence le DIPA ou IPM ;
- les isoparaffines C13 à C40 ;
- les paraffines liquides ;
- la gelée de pétrole ;
et des combinaisons de ceux-ci.

5. Gel anhydre transparent selon l'une quelconque des revendications 1 à 4, **caractérisé en ce que** les substances non volatiles comprennent 0 à 2 % (en poids des substances non volatiles) d'au moins un composé qui est non volatile, soluble dans les substances volatiles et non soluble dans les polymères de silicone fonctionnalisés utilisés pour la préparation du gel et qui sera réticulé par un agent gélifiant pour obtenir lesdits polymères de silicone réticulés, et des combinaisons de ceux-ci.

6. Gel anhydre transparent selon la revendication 5, **caractérisé en ce que** ledit composé est choisi dans le groupe comprenant le dipropylèneglycol, le propylèneglycol, le benzoate de benzyle, le phtalate de diéthyle, le citrate de triéthyle, de préférence le dipropylèneglycol ou le citrate de triéthye.

7. Gel anhydre transparent selon l'une quelconque des revendications 1 à 6, **caractérisé en ce que** la turbidité du gel est inférieure à 20 NTU, de préférence inférieure à 12 NTU, de manière préférée entre toutes inférieure à 8 NTU.

8. Gel anhydre transparent selon l'une quelconque des revendications 1 à 7, **caractérisé en ce que** la résistance de gel relative du gel est inférieure à 10 g/mm.

9. Produit comprenant un gel anhydre transparent selon l'une quelconque des revendications 1 à 8, inclus dans un récipient.

10. Procédé de production d'un gel anhydre transparent présentant une résistance de gel relative inférieure à 20 g/mm, selon l'une quelconque des revendications 1 à 8, comprenant les étapes suivantes :
(a) le mélange d'une ou plusieurs substances volatiles et d'une ou plusieurs substances non volatiles, lesdites une ou plusieurs substances non volatiles comprennent 3,77 % à 100 % (en poids des substances non volatiles) de polymères de silicone fonctionnalisés et 0 à 96,23 % (en poids des substances non volatiles) d'un solvant non volatil et lesdites une ou plusieurs substances volatiles comprennent 20,6 à 100 % (en poids des substances volatiles) de parfum et 0 à 80 % (en poids des substances volatiles) de solvant volatil, et le rapport de substance volatile / polymères de silicone fonctionnalisés est compris entre 1 et 32 ;
(b) l'ajout d'un agent gélifiant au mélange obtenu après l'étape (a), ledit agent gélifiant représentant 0,025 à 3,5 % en poids du mélange obtenu après l'étape (a) ;
aucune charge ni aucun agent renforçant n'étant ajouté à l'étape (a) ou (b).

11. Procédé selon la revendication 10, **caractérisé en ce que** la fonction desdits polymères de silicone fonctionnalisés est choisie dans le groupe comprenant un groupe vinyle, un groupe allyle, un groupe acryle, un groupe hydroxyle, et un groupe Si-H.

12. Procédé selon la revendication 10 ou 11, **caractérisé en ce que** lesdits polymères de silicone fonctionnalisés sont choisis dans le groupe comprenant un polydiméthylsiloxane inhibé à terminaison hydroxyle, présentant de préférence une viscosité comprise entre 40 et 3 000 000 mm²/s.

13. Procédé selon l'une quelconque des revendications 10 à 12, **caractérisé en ce que** les étapes, en particulier l'étape (b), sont réalisées à température ambiante.

14. Procédé selon l'une quelconque des revendications 11 à 13, **caractérisé en ce que** 0,025 à 3,5 % en poids du mélange obtenu après l'étape (a) de l'agent gélifiant consistent en :
- 0,2 à 2,5 % en poids du mélange obtenu après l'étape (a) d'agent réticulant, de préférence le tétraalcoxysilane ou le silane ;
- 0,05 à 1 % en poids du mélange obtenu après l'étape (a) de catalyseur, de préférence le titane, le platine ou l'étain, de manière davantage préférée l'étain.

15. Kit permettant d'obtenir un gel anhydre transparent présentant une résistance de gel relative inférieure à 20 g/mm, selon la présente invention et selon l'une quelconque des revendications 1 à 8, comprenant :
(i) une ou plusieurs substances volatiles et une ou plusieurs substances non volatiles, lesdites une ou plusieurs substances non volatiles comprennent 3,77 % à 100 % (en poids des substances non volatiles) de polymères de silicone fonctionnalisés et 0 à 96,23 % (en poids des substances non volatiles) d'un solvant non volatil et lesdites une ou plusieurs substances volatiles comprennent 20,6 à 100 % (en poids des substances volatiles) de parfum et 0 à 80 % (en poids des substances volatiles) de solvant volatil, et le rapport de substance volatile / polymères de silicone fonctionnalisés est compris entre 1 et 32 ;
(ii) un agent gélifiant,
où (ii) est 0,025 à 3,5 % en poids de (i), (ii) est fourni séparément du mélange (i), et le kit ne comprend pas de charge ou d'agent renforçant.
